# EUROPÄISCHE PATENTSCHRIFT

(11) **EP 3 361 253 B1**
(45) Veröffentlichungstag und Bekanntmachung des Hinweises auf die Patenterteilung: **04.12.2019**
(21) Anmeldenummer: 17155143.5
(22) Anmeldetag: 08.02.2017
(51) Int. Cl.: G01N 33/53, G01N 21/84

(54) **VORRICHTUNG ZUR GEZIELTEN PRÜFUNG UND ANZEIGE DER AB- BZW. ANWESENHEIT VON ANALYTEN IN EINER FLÜSSIGEN PROBE**
DEVICE FOR THE TARGETED TESTING AND INDICATION OF THE ABSENCE OR PRESENCE OF ANALYTES IN A LIQUID SAMPLE
DISPOSITIF DE CONTRÔLE CIBLÉ ET D'AFFICHAGE DE LA PRÉSENCE OU DE L'ABSENCE D'ANALYTES DANS UN ÉCHANTILLON LIQUIDE

(43) Veröffentlichungstag der Anmeldung: 15.08.2018
(73) Patentinhaber: Protzek Gesellschaft für Biomedizinische Technik GmbH, 4133 Prattein (CH)
(72) Erfinder: PROTZEK, Christoph, 79539 Lörrach (DE)
(74) Vertreter: Patentanwälte Dörner & Kötter PartG mbB

(56) Entgegenhaltungen:
- EP-A1- 2 420 831
- WO-A1-2014/043247
- WO-A1-2016/064545
- DE-U1-202016 104 645
- US-A1- 2007 092 401
- US-A1- 2008 299 010
- Patricia Zane ET AL: "Dried blood spots: the future" In: "Microsampling in Pharmaceutical Bioanalysis", Dezember 2013 (2013-12), Future Science Ltd, Unitec House, 2 Albert Place, London N3 1QB, UK, XP055382813, ISBN: 978-1-909453-58-6 Seiten 48-66, DOI: 10.4155/ebo.13.442, * Abbildung 4.2. *
- EDELBROEK PETER M ET AL: "Dried blood spot methods in therapeutic drug monitoring: methods, assays, and pitfalls", THERAPEUTIC DRUG MONITOR, LIPPINCOTT WILLIAMS AND WILKINS, NEW YORK, NY, US, Bd. 31, Nr. 3, Juni 2009 (2009-06), Seiten 327-336, XP009126006, ISSN: 0163-4356

## Beschreibung

Die Erfindung betrifft eine Vorrichtung zur gezielten Prüfung und Anzeige der Ab- bzw. Anwesenheit von Analyten in einer vom menschlichen oder tierischen Organismus stammenden oder sonstigen Analysen- oder Flüssigkeitsprobe nach dem Oberbegriff des Patentanspruchs 1.

Vorrichtungen zur Prüfung und Anzeige der Ab- bzw. Anwesenheit von Analyten in einer flüssigen Probe, z.B. von Wirkstoffen wie Sucht- oder Arzneistoffen und ihren Stoffwechselprodukten oder von biologischen Stoffen als Zeichen (Markern) für bestimmte Erkrankungen in einer Körperflüssigkeit sind in unterschiedlichen Ausführungen bekannt. Dabei handelt es sich in der Regel um in relativ kurzer Zeit ablaufende Analysensysteme auf immunologischer oder auch trockenchemischer Basis, wie auch DNA basiert (sogenannte Aptamere). Diese sind jedoch aufgrund ihrer Funktionsweise nicht vollkommen beweisend im forensischen Sinn. Die Analyseprinzipien bestehen in einer mehr oder minder hochspezifischen Reaktion auf die ggf. anwesenden Analyten. Das bedeutet, dass in seltenen, nicht vorhersehbaren Fällen auch durch zuvor unbekannte oder unspezifische Störfaktoren in einer Probe eine ähnliche bis vergleichbare Reaktion verursacht werden kann. Aus diesem Grund sind immunologische Tests sogenannte vorläufige Analysen, die im Bedarfsfall zum Beleg ihrer Richtigkeit bzw., falls das Ergebnis Rechtskraft bekommen soll, durch eine bislang laborgebundene beweisende Analyse (Bestätigungsanalyse), bspw. mittels massenspektrometischer Analysetechniken zu ergänzen sind.

Besonderes Interesse besteht an solchen Geräten, die auch außerhalb eines Labors eingesetzt werden können. Diese müssen tragbar und leicht zu bedienen sein sowie in kurzer Zeit ein zuverlässiges Ergebnis liefern. Insbesondere zur Durchführung von Tests auf Drogenkonsum oder Medikamentenmissbrauch oder dergleichen durch die Polizei ist eine Vorrichtung erforderlich, die auch im Freien bei unterschiedlichen wechselnden Lichtverhältnissen, Wetterverhältnissen und Temperaturen einen derartigen Test ermöglicht. In der DE 20 2014 002 369 U1 wird hierzu ein Gerät vorgeschlagen, in das eine Testkassette einführbar ist und das mit einer Lichtquelle versehen ist, mittels derer die untere Wand der aus einem lichtdurchlässigem Kunststoff bestehenden Testkassette sowie den Testbereich des in der Testkassette angeordneten Teststreifens durchstrahlt und das Testergebnis ausleuchtet. Die Testkassette ist zur Durchführung eines sogenannten "lateral flow"- Immunoassays ausgelegt und besteht aus einem unteren Deckel und einem oberen Deckel. Eingeschlossen von diesen beiden Deckeln enthält die Testkassette einen Teststreifen, der innerhalb einer Aufnahme in Position gehalten wird. In dem oberen Deckel befindet sich über dem Probenaufnehmer des Teststreifens eine Einfüllöffnung, durch die eine flüssige Probe eingegeben werden kann. Über dem mittleren Testbereich des Teststreifens befindet sich ein Kontrollfenster, durch welches das Testergebnis abgelesen werden kann.

In der DE 20 2016 104 645 U1 ist eine Vorrichtung zum gezielten Prüfung auf Ab- bzw. Anwesenheit von Analyten in einer flüssigen Probe beschrieben, die gegenüber den zuvor beschriebenen Kassetten einfacher aufgebaut ist, für den mobilen Einsatz geeignet ist und die nur wenig Platz beansprucht. Hierzu umfasst die Vorrichtung eine Trägerplatte mit Teststreifenaufnahmen, in denen jeweils ein mit einem Probeaufnahmeabschnitt und einem Testabschnitt versehener Teststreifen angeordnet ist, und die über eine nicht lösbar mit der Trägerplatte verbundene Deckschicht verschlossen ist, die im Bereich der Testabschnitte der Teststreifen der Teststreifenaufnahmen ein Kontrollfenster aufweist. Derartige Vorrichtungen haben sich im mobilen Einsatz bewährt. Dabei hat sich als besonders vorteilhaft eine Gestaltung erwiesen, bei der die Trägerplatte aus Pappe hergestellt ist, wobei die Folie aus verrottbarem Material gebildet ist, da solche Vorrichtungen umweltschonend zu entsorgen sind.

Nach wie vor erweist sich im Falle von Blutanalysen auch bei den Vorrichtungen der vorgenannten Art die erforderliche, begleitende Blutentnahme als aufwendig, da hierzu u.a. ein Blutentnahmeset erforderlich ist. Insbesondere für rechtliche Zwecke wird dem menschlichen Organismus entnommenes Blut in einem Röhrchen deponiert, das zusammen mit der Vorrichtung einer Bestätigungsanalyse zugeleitet wird. Hierbei ist insbesondere zu beachten, dass sich in der Probe zur Zeit der Blutabnahme möglicherweise vorhandene instabile Analyte innerhalb des Röhrchens im Zeitraum bis zum Eintreffen im Labor bzw. bis zur Ausführung der Bestätigungsanalyse verändern können.

Hier will die Erfindung Abhilfe schaffen. Der Erfindung liegt die Aufgabe zu Grunde, eine Vorrichtung zur gezielten Prüfung und Anzeige der Ab- bzw. Anwesenheit von Analyten in einer flüssigen Probe bereitzustellen, die einfach aufgebaut ist und bei der eine aufwendige Blutentnahme nicht erforderlich ist. Gemäß der Erfindung wird diese Aufgabe durch eine Vorrichtung mit den Merkmalen des Patentanspruchs 1 gelöst.

In der EP2420831 wird ein "dried spot" Probenträger (DS Probenträger) offenbart.

Mit der Erfindung ist eine Vorrichtung zur gezielten Prüfung und Anzeige der Ab- bzw. Anwesenheit von Analyten in einer flüssigen Probe bereitgestellt, die einfach aufgebaut ist und bei der die im Stand der Technik erforderliche, aufwendige Blutentnahme nicht erforderlich ist. Dadurch, dass beabstandet zu der Teststreifenaufnahme wenigstens eine Probenträgeraufnahme angeordnet ist, die einen "dried spot" - Probentröger (DS-Probenträger) aufnimmt, können auf der Vorrichtung selbst einige wenige Tropfen einer Probenflüssigkeit (z.B. Blutstropfen) bevorratet werden. Die definierten eingetrockneten Flüssigkeitsrückstände können sodann im Falle von Blut als sogenannte "dried blood spots" (DBS), im Falle von Urin als sogenannte "dried urine spots" (DUS) oder im Falle von Speichel als sogenannte "dries saliva spots" (DSS) etc., gemeinsam mit den Teststreifen zur Bestätigungsanalyse gegeben werden. In der Vorrichtung sind somit bereits alle für eine Bestätigungsanalyse benötigten Proben integriert, wie auch der vorangegangene Vortest, der den Archivierungsprozess für den DBS/DSS/DUS ausgelöst hat.

Die DS-Analyse hat sich in der Handhabung von Mikroprobemengen seit Langem etabliert. Bei Analysen für rechtliche Zwecke besteht ein Vorteil insbesondere auch darin, dass Enzymaktivitäten, die ursprünglich vorhandene Stoffe aufbauen oder umwandeln können, infolge des raschen Verlusts ihrer Hydrathülle weitgehend eingeschränkt werden. Bereits wenige Blutstropfen, die aus der Fingerbeere oder der Ferse entnommen werden können, genügen, um eine DBS-Analyse durchzuführen. Bevorzugt weist der DS-Probenträger wenigstens drei, vorzugsweise vier bevorzugt kreisförmig ausgebildete markierte Bereiche auf. Die Blutstropfen werden manuell per Lanzette, Pipette etc. in diese kreisförmig markierten Bereiche auf den Probenträger aufgebracht und zwar in einer definierten Menge, die sicherstellt, dass sich das Blut in dem durch Striche definierten Bereich homogen verteilt. Dabei kann die Beaufschlagung unmittelbar am Ort der Probenahme erfolgen. Sobald das Blut eingetrocknet ist, lässt sich der DBS-Probenträger sofort einer Analyse zuführen. Aufgrund der homogen Verteilung der Bestandteile und Inhaltsstoffe innerhalb des Blutstropfens ist es möglich, durch Ausstanzen einer Scheibe definierten Durchmessers aus dem DBS-Probenträger ein ebenfalls definiertes Blutvolumen zu entnehmen und zu untersuchen. Gleiches gilt auch für andere homogene Analyseflüssigkeiten, wie Urin und Speichel oder geeignete Lösungen von Stoffproben.

Bevorzugt ist der DS-Probenträger aus Cellulose hergestellt. Je nach Einsatzfeld können jedoch auch weitere Materialien, z.B. ein DS-Probenträger auf Polymerbasis eingesetzt werden, die gewisse Funktionalitäten erfüllen und etwa die Möglichkeit bieten, Proteine zu fällen. Besonders bevorzugt ist der DS-Probenträger in Form einer Cellulosekarte oder eines Filterpapiers ausgebildet.

In Weiterbildung der Erfindung ist der DS-Probenträger zumindest bereichsweise über eine nicht lösbar mit der Trägerplatte verbundenen Deckschicht verschlossen, welche Deckschicht im Bereich der markierten Bereiche wenigstens eine Öffnung aufweist, die über eine lösbare Abdeckung verschlossen ist. Hierdurch ist eine Kontaminierung des DS-Probenträgers vor dem Gebrauch verhindert. Vor Aufgabe einer Probe auf den DS-Probenträger kann die lösbare Abdeckung entfernt werden.

In weiterer Ausgestaltung der Erfindung ist die lösbare Abdeckung durch eine in der Deckschicht eingebrachte Perforationslinie begrenzt. Die Freigabe des DS-Probenträgers erfolgt hier durch Heraustrennung der Abdeckung entlang der Perforationslinie.

In Ausgestaltung der Erfindung ist wenigstens eine Teststreifenaufnahme zumindest bereichsweise über eine nicht lösbar mit der Trägerplatte verbundene Deckschicht verschlossen, welche Deckschicht im Bereich des Testsabschnitts des Teststreifens der wenigstens einen Teststreifenaufnahme ein Kontrollfenster aufweist, wobei die Deckschicht ein zu dem Kontrollfenster beabstandet angeordnetes Probeaufgabefenster aufweist, das eine Probeaufgabe auf den Probeaufnahmeabschnitt wenigstens eines Teststreifens ermöglicht. Hierdurch ist eine sehr platzsparende, komprimierte Bauweise erzielt.

In Weiterbildung der Erfindung sind wenigstens zwei parallel zueinander angeordnete, jeweils einen Teststreifen aufnehmende Teststreifenaufnahmen angeordnet, die an einem Ende in einem gemeinsamen Reservoir münden, in dem die Probeaufnahmeabschnitte der Teststreifen angeordnet sind und über dem das Probeaufgabefenster der Deckschicht positioniert ist. Hierdurch ist eine gleichzeitige Probeaufgabe auf alle in dem Reservoir mündenden Probeaufnahmeabschnitte der Teststreifen ermöglicht. Über die Ausgestaltung des Reservoirs ist zudem eine ausreichende Zufuhr an Flüssigkeitsprobe gewährleistet.

In weiterer Ausgestaltung der Erfindung ist die Trägerplatte aus Pappe hergestellt, wobei vorhandene Teststreifenaufnahmen und/oder Reservoire und/oder Probenträgeraufnahmen durch Einprägungen ausgebildet sind. Hierdurch ist eine umweltschonende Entsorgung der Vorrichtung erzielt. In alternativer Ausgestaltung der Erfindung können vorhandene Teststreifenaufnahmen und/oder Reservoire und/oder Probenträgeraufnahmen auch durch Ausnehmungen einer zwischen Deckfolie und Trägerplatte angeordneten Zwischenschicht ausgebildet sein.

In Weiterbildung der Erfindung ist die Deckschicht aus einer Folie gebildet, die bevorzugt im Wesentlichen flächig mit der Trägerplatte und den von der Trägerplatte aufgenommenen Teststreifen sowie DS-Probeträgern bereichsweise verklebt ist. Hierdurch ist ein Schutz der Teststreifen sowie der DS-Probenträger vor Kontamination erzielt. Dabei ist die Folie vorteilhaft aus verrottbarem Material hergestellt. Das wenigstens eine Kontrollfenster ist vorteilhaft durch jeweils einen transparenten Bereich der Folie gebildet.

In weiterer Ausgestaltung der Erfindung sind zwei schwenkbar miteinander verbundene Trägerplatten angeordnet, wobei eine Trägerplatte mit wenigstens einer Teststreifenaufnahme und die an dieser anliegende andere Trägerplatte mit wenigstens einer Probenträgeraufnahme versehen ist. Dabei sind bevorzugt die wenigstens eine Teststreifenaufnahme sowie die wenigstens eine Probenträgeraufnahme jeweils auf einer Hälfte der Oberfläche einer Trägerplatte angeordnet, derart, dass im eingeklapptem Zustand jeweils die mit Teststreifenaufnahmen versehene Hälfte einer Trägerplatte an der keine Probenträgeraufnahme aufweisenden Hälfte der an dieser anliegenden anderen Trägerplatte anliegt. Hierdurch ist eine Anordnung von Teststreifen und DS-Probenträger auf beiden Seiten der Vorrichtung erzielt, wodurch eine kompakte, dünne Bauweise der Vorrichtung bewirkt ist.

In weiterer Ausgestaltung der Erfindung ist wenigstens ein Reservoir und/oder eine Probenträgeraufnahme zumindest bereichsweise von punkt- oder linienförmigen, aus der Trägerplatte heraustretenden Erhebungen eingefasst. Hierdurch ist die Oberflächenspannung der umgebenden Bereiche beim Befüllen erhöht, so dass ein Überlaufen der aufzutragenden Flüssigkeit verhindert ist. Dabei sind die Erhebungen bevorzugt als punktförmige Nippel, wie sie bei der Blindenschrift Verwendung finden, ausgeführt.

Andere Weiterbildungen und Ausgestaltungen der Erfindung sind in den übrigen Unteransprüchen angegeben. Ein Ausführungsbeispiel der Erfindung ist in den Zeichnungen dargestellt und wird nachfolgend im Einzelnen beschrieben. Es zeigen:

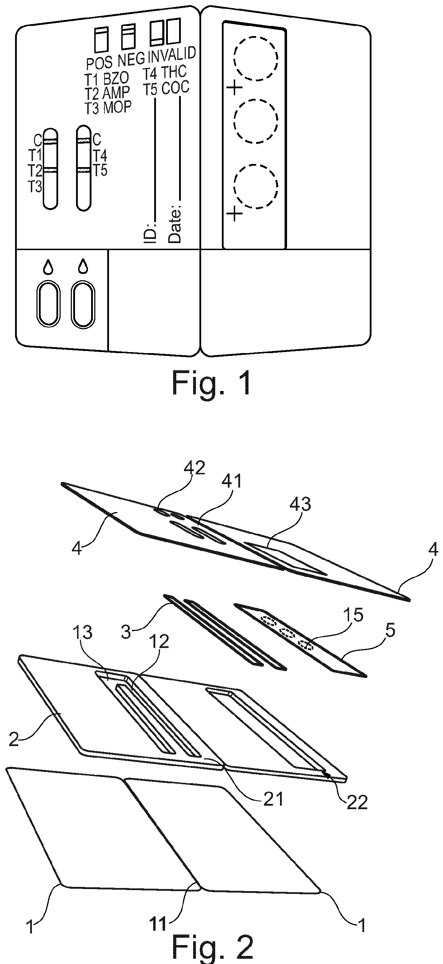

- Figur 1: die schematische Darstellung einer Vorrichtung zum visuellen Nachweis von Analyten in einer flüssigen Probe;
- Figur 2: die Darstellung der Vorrichtung aus Figur 1 in Explosionsdarstellung;
- Figur 3: die schematische Darstellung eines Teststreifens der Vorrichtung aus Figur 1 und
- Figur 4: die schematische Darstellung eines DS-Probenträgers der Vorrichtung aus Figur 1.

Die als Ausführungsbeispiel gewählte Vorrichtung zur gezielten Prüfung und Anzeige der Ab- bzw. Anwesenheit von Analyten in einer flüssigen Probe besteht im Wesentlichen aus zwei entlang ihrer Längsachse schwenkbar miteinander verbundenen Trägerplatten 1, auf denen jeweils eine Zwischenschicht 2 aufgebracht ist, die zwei Teststreifen 3 sowie einen DS-Probenträger 5 aufnimmt und die mit einer Deckschicht 4 versehen ist.

Die Trägerplatten 1 sind im Ausführungsbeispiel aus Pappe hergestellt und aus einem einzigen mittig mit einer Knickfalz 11 versehenen Kartonzuschnitt gebildet.

Die Zwischenlage 2 ist im Ausführungsbeispiel aus Wellpappe hergestellt und mit einer transparenten, flüssigkeitsdichtenden Folie beschichtet, um ein Eindringen von Probenflüssigkeit in den Karton zu verhindern. Alternativ kann die Zwischenschicht 2 auch mit einem wasserdichten, vorzugsweise umweltgerechten Lack versehen sein. In der Zwischenschicht 2 ist eine Ausnehmung 21 eingebracht, durch die jeweils zwei Teststreifenaufnahmen 12 gebildet sind, die in einem Reservoir 13 münden. Die Teststreifenaufnahmen 12 nehmen die Teststreifen 3 auf. Weiterhin ist in der Zwischenschicht eine zweite Ausnehmung 22 vorhanden, durch die eine Probenträgeraufnahme 14 gebildet ist, die den DS-Probenträger 5 aufnimmt.

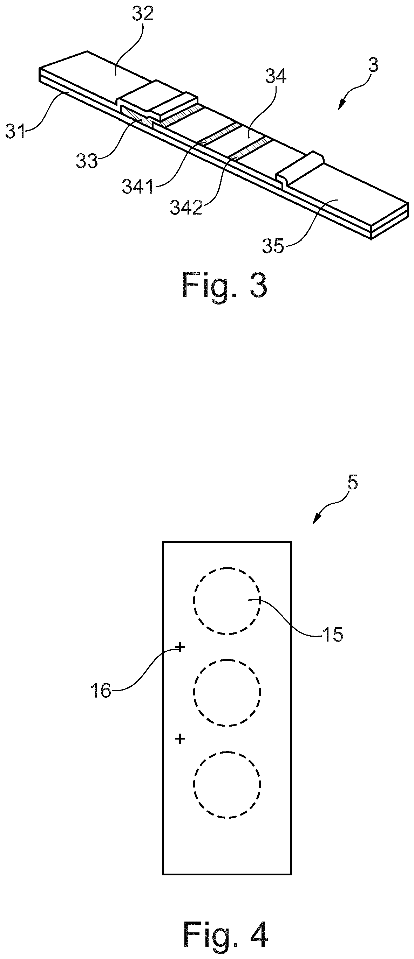

Der Teststreifen 3 ist in Figur 3 im Stufenschnitt dargestellt. Der Teststreifen 3 weist eine Trägerfolie 31 auf, auf der an einem Ende ein Probeaufnahmeabschnitt 32 angeordnet ist. Unmittelbar benachbart und teilweise unter dem Probeaufnahmeabschnitt 32 befindet sich auf der Trägerfolie 31 ein Antikörperbelag 33, der Antikörper enthält, die mit Goldpartikeln konjugiert sind und die zu einem festzustellenden Analyten bzw. zu einem Antigen dieses Analyten passen und in eine immunologische Reaktion treten können. An den Antikörperbelagabschnitt 33 schließt sich ein Testabschnitt 34 an, der in Form einer Membran ausgebildet ist, auf den sich beabstandet zueinander eine Testlinie 341 und eine Kontrolllinie 342 befinden, die durch in einem Reagenz für den Nachweis eines bestimmten Analyten enthaltenen Antigen ausgebildet sind und vor Aufgabe einer Probe unsichtbar sind. Der Probenaufnahmeabschnitt 32 des Teststreifens 3 befindet sich unterhalb der Probenaufgabefenster 42 und der Testabschnitt 34 befindet sich unterhalb des Kontrollfensters 41 der auf der Zwischenschicht 2 aufgebrachten Deckschicht 4.

Bei der Durchführung eines "lateral flow" Immunoassays zeigt die Testlinie 341 das Ergebnis des Tests an; die Kontrolllinie 342 gibt an, ob eine ausreichende Probemenge aufgegeben worden ist, also ob der Test valide ist. An seinem dem Probenaufnahmeabschnitt 32 gegenüberliegenden Ende ist an den Teststreifen 3 ein Absorberabschnitt 35 angeordnet.

Der DS-Probenträger 5 ist in Form einer Cellulose-Karte ausgebildet, welche mit drei kreisförmig markierten Probeaufgabebereichen 15 versehen ist. Weiterhin weist der DS-Probenträger 5 zwei kreuzförmige Positioniermarkierungen 16 auf. Die Positioniermarkierungen 16 dienen der lagegerechten Zuführung des DS-Probenträgers 5 zu einer Ausstanzvorrichtung, mittels der aus dem DS-Probenträger 5 eine Scheibe definierten Durchmessers ausgestanzt werden kann.

Die Deckschicht 4 ist im Ausführungsbeispiel aus einer verrottbaren, farbigen Folie gebildet, die mit transparent ausgebildeten, klaren, reflektionsarmen Kontrollfenstern 41 versehen ist. Beabstandet zu den Kontrollfenstern 41 ist ein Probeaufgabefenster 42 angeordnet, das durch eine in der Deckschicht 4 eingebrachte Ausnehmung gebildet ist. Das Probeaufgabefenster 42 ist derart angeordnet, dass es über den Probeaufnahmeabschnitten 32 beider Teststreifen 3 positioniert ist. Im Ausführungsbeispiel ist zwischen den Teststreifen 3 und dem Probeaufgabefenster 42 der Deckschicht 4 ein - nicht dargestelltes - Probeaufnahmevlies angeordnet, das die Probenaufnahmeabschnitte 32 der beiden Teststreifen 3 überdeckt. Weiterhin ist in der Deckschicht 4 eine Ausnehmung 43 angeordnet, die einen Zugang zu dem DS-Probenträger 5 ermöglicht. Die Ausnehmung 43 ist im Ausführungsbeispiel über eine - nicht dargestellte - lösbare Abdeckung verschlossen.

Vor der Durchführung eines Tests sind keine Linien 341, 342 ersichtlich. Bei der Durchführung eines Tests wird ein Probevolumen einer Probeflüssigkeit durch ein Probeaufgabefenster 42 der Deckschicht 4 auf den Probenaufnahmeabschnitt 32 eines jeden Teststreifens 3 aufgegeben. Durch Kapilarwirkung fließt die Probenflüssigkeit in Richtung Testabschnitt 34. Sie kommt dabei zunächst mit dem Antikörperbelagabschnitt 33 in Kontakt, in dem sich mit Goldpartikeln konjugierte Antikörper zum Antigen eines festzustellenden Analyten in standardisierter (dosierter) Menge befinden. Die Antikörper weisen eine für verschiedene Analyten (Drogen) unterschiedliche Bindungsaktivität auf. Die mit Goldpartikeln konjugierten Antikörper werden durch die Probenflüssigkeit mitgenommen, welche den Testabschnitt 34 durchfließt. Enthält die Probenflüssigkeit den festzustellenden Analyten nicht bzw. weniger als die im Test angelegte Anzeigegrenze, so binden beim Durchfließen des Testsabschnitts 34 die dort im Bereich der Testlinie 341 befindlichen Antigene des Analyten die von der Probenflüssigkeit mitgenommenen Antikörper. Es bildet sich daraufhin eine sichtbare Testlinie 341 heraus, die durch die mit den Antikörpern konjugierten Goldpartikel eine rote oder braune Farbe erhält. Der Testbefund ist damit negativ.

Enthält die Probenflüssigkeit den festzustellenden Analyten, so wird dieser als Antigen durch die Antikörper bereits im Bereich des Antikörperbelagabschnitts 33 des Teststreifens 3 gebunden und es kann im Testabschnitt 34 nicht mehr zu einer Anlagerung kommen, weshalb sich keine sichtbare Testlinie 341 herausbildet. Das Testergebnis ist in diesem Falle positiv. Die Kontrolllinie 342 zeigt an, ob der Test valide ist. In Figur 3 ist die Testlinie 341 farbig herausgebildet, weshalb der Test in Bezug des entsprechenden Analyten negativ ist.

Im Rahmen der Durchführung des Tests wird zugleich auf die Probenaugabenbereiche 15 des DS-Probenträgers eine geringe Menge weiteren biologischen Materials, beispielsweise Blut, Urin oder Speichel definiert aufgetragen. Ergibt sich bei der Diagnose der Teststreifen 3 ein positiver Befund, so kann mit dem in den Probenaufgabebereichen 15 deponierten biologischen oder sonstigen Material eine Bestätigungsanalyse durchgeführt werden, beispielsweise mit Hilfe von Flüssigkeits- (Liquid-) Chromatographie-Tandem-Massenspektrometrie (LC-MS/MS). Vorteilhaft dabei ist, dass das biologische Material bei der Probenahme fixiert ist und nicht mehr manipuliert werden kann und dass sich etwaige Reaktionen, die den ursprünglichen Probenzustand beeinträchtigen, wie beispielsweise durch Ester-spaltende Enzyme etc., nicht mehr stattfinden. Des Weiteren entfällt bei dieser Arbeitsweise der vollständige Probenversand per Urin-, Blut- oder Speichelröhrchen und damit die im Stand der Technik gängigen Blutentnahme-Sets, wodurch erhebliche Kosten verursacht sind.

Die erfindungsgemäße Vorrichtung zeichnet sich dadurch aus, dass sie bei einer kompakten Ausbildung sowohl einen Test von einer großen Anzahl von Analyten, sowie auch zugleich die Deponierung von weiterem biologischen Materials, insbesondere Blut, Urin, Speichel oder anderen zu analysierenden Flüssigkeitsproben ermöglicht. Die erfindungsgemäße Vorrichtung kann somit im Rahmen eines Tests mit allen für eine Bestätigungsanalyse erforderlichen Proben versehen werden. Dabei sind die von den Probeaufgabebereichen 15 aufgenommenen Proben im Nachhinein nicht mehr veränderbar. Durch die Herstellung der Vorrichtung im Wesentlichen aus Kartonwerkstoffen ist zudem eine umweltgerechte Entsorgung dieser zur einmaligen Verwendung bestimmten Vorrichtung ermöglicht.

## Patentansprüche

1. Vorrichtung zur Prüfung und Anzeige der An- bzw. Abwesenheit von Analyten in einer vom menschlichen oder tierischen Organismus stammenden Analysen- oder Flüssigkeitsprobe, umfassend eine Trägerplatte mit wenigstens einer Teststreifenaufnahme, in der ein mit einem Probeaufnahmeabschnitt und einem Testabschnitt versehener Teststreifen angeordnet ist, **dadurch gekennzeichnet, dass** die Trägerplatte beabstandet zu der Teststreifenaufnahme (12) wenigstens eine Probenträgeraufnahme (14) umfasst, wobei die Vorrichtung weiterhin einen "dried spot" Probenträger (DS-Probenträger) (5) beinhaltet, der in der Probenträgeraufnahme (14) aufgenommen ist.

2. Vorrichtung nach Anspruch 1, **dadurch gekennzeichnet, dass** der DS-Probenträger (5) wenigstens drei, vorzugsweise vier bevorzugt kreisförmig ausgebildete markierte Probenaufgabebereiche (15) aufweist.

3. Vorrichtung nach Anspruch 1 oder 2, **dadurch gekennzeichnet, dass** der DS-Probenträger (5) aus Cellulose hergestellt ist.

4. Vorrichtung nach Anspruch 3, **dadurch gekennzeichnet, dass** der DS-Probenträger (5) in Form eines Filterpapiers ausgebildet ist.

5. Vorrichtung nach einem der Ansprüche 2 bis 4, **dadurch gekennzeichnet, dass** der DS-Probenträger (5) zumindest bereichsweise über eine nicht lösbar mit der Trägerplatte (1) verbundenen Deckschicht (4) verschlossen ist, welche Deckschicht (4) im Bereich der markierten Probenaufgabebereiche (15) wenigstens eine Ausnehmung (43) aufweist, die über eine lösbare Abdeckung verschlossen ist.

6. Vorrichtung nach Anspruch 5, **dadurch gekennzeichnet, dass** die lösbare Abdeckung durch eine in der Deckschicht (4) eingebrachte Perforationslinie begrenzt ist.

7. Vorrichtung nach einem der vorgenannten Ansprüche, **dadurch gekennzeichnet, dass** wenigstens eine Teststreifenaufnahme (12) zumindest bereichsweise über eine nicht lösbar mit der Trägerplatte (1) verbundenen Deckschicht (4) verschlossen ist, welche Deckschicht (4) im Bereich des Testabschnitts (34) des Teststreifens (3) der wenigstens einen Teststreifenaufnahme (12) ein Kontrollfenster (41) aufweist, wobei die Deckschicht (4) ein zu dem Kontrollfenster (41) beabstandet angeordnetes Probeaufgabenfenster (42) aufweist, das eine Probeaufgabe auf den Probeaufnahmeabschnitt (32) wenigstens eines Teststreifens (3) ermöglicht.

8. Vorrichtung nach Anspruch 7, **dadurch gekennzeichnet, dass** wenigstens zwei parallel zueinander angeordnete, jeweils einen Teststreifen (3) aufnehmende Teststreifenaufnahmen (12) angeordnet sind, die an einem Ende in einem gemeinsamen Reservoir (13) münden, in dem die Probeaufnahmeabschnitte (32) der Teststreifen (3) angeordnet sind und über dem das Probeaufgabefenster (42) der Deckschicht (4) positioniert ist.

9. Vorrichtung nach einem der vorgenannten Ansprüche, **dadurch gekennzeichnet, dass** die Trägerplatte (1) aus Pappe hergestellt ist, wobei vorhandene Teststreifenaufnahmen (12) und/oder Reservoire (13) und/oder Probenträgeraufnahmen (14) durch Einprägungen ausgebildet sind.

10. Vorrichtung nach einem der Ansprüche 1 bis 8, **dadurch gekennzeichnet, dass** vorhandene Teststreifenaufnahmen (12) und/oder Reservoire (13) und/oder Probenträgeraufnahmen (14) durch Ausnehmungen (21) einer zwischen Deckfolie (4) und Trägerplatte (1) angeordneten Zwischenschicht (2) ausgebildet sind.

11. Vorrichtung nach einem der vorgenannten Ansprüche, **dadurch gekennzeichnet, dass** die Deckschicht (4) aus einer Folie gebildet ist, die bevorzugt im Wesentlichen flächig mit der Trägerplatte (1) und den von der Trägerplatte (1) aufgenommenen Teststreifen (3) sowie mit dem DS-Probenträger (5) verklebt ist.

12. Vorrichtung nach einem der vorgenannten Ansprüche, **dadurch gekennzeichnet, dass** zwei schwenkbar miteinander verbundene Trägerplatten (1) angeordnet sind, wobei eine Trägerplatte mit wenigstens einer Teststreifenaufnahme (12) und die an dieser anliegende andere Trägerplatte (1) mit wenigstens einer Probenträgeraufnahme (14) versehen ist.

13. Vorrichtung nach Anspruch 12, **dadurch gekennzeichnet, dass** die wenigstens eine Teststreifenaufnahme (12) sowie die wenigstens eine Probenträgeraufnahme (14) jeweils auf einer Hälfte der Oberfläche einer Trägerplatte (1) angeordnet sind, derart, dass im eingeklappten Zustand jeweils die mit Teststreifenaufnahmen (12) versehene Hälfte einer Trägerplatte (1) an der keine Probenträgeraufnahme (14) aufweisenden Hälfte der an dieser anliegenden anderen Trägerplatte (1) anliegt.

14. Vorrichtung nach einem der vorgenannten Ansprüche, **dadurch gekennzeichnet, dass** wenigstens ein Reservoir (13) und/oder eine Probenträgeraufnahme (14) zumindest bereichsweise von punkt- und/oder linienförmigen, aus der Trägerplatte (1) heraustretenden Erhebungen eingefasst sind.

## Claims

1. Device for the testing and indication of the absence or presence of analytes in an analysis or liquid sample originating from the human or animal organism comprising a carrier plate with at least one test strip receptacle, in which a test strip provided with a sample receiving section and a test section is arranged, **characterised in that** the carrier plate comprises at least one sample carrier receptacle (14) spaced apart from the test strip receptacle (12), wherein the device further includes a "dried spot" sample carrier (DS sample carrier) (5), which is received in the sample carrier receptacle (14).

2. Device according to claim 1, **characterised in that** the DS sample carrier (5) has at least three, preferably four marked sample application areas (15) preferably of circular construction.

3. Device according to claim 1 or 2, **characterised in that** the DS sample carrier (5) is made of cellulose.

4. Device according to claim 3, **characterised in that** the DS sample carrier (5) is designed in the form of a filter paper.

5. Device according to one of claims 2 to 4, **characterised in that** the DS sample carrier (5) is at least partially closed via a cover layer (4) non-detachably connected to the carrier plate (1), which cover layer (4) has at least one recess (43) in the area of the marked sample application areas (15), which is closed via a detachable cover.

6. Device according to claim 5, **characterised in that** the detachable cover is delimited by a perforation line inserted in the cover layer (4).

7. Device according to one of the previous claims, **characterised in that** at least one test strip receptacle (12) is at least partially closed via a cover layer (4) non-detachably connected to the carrier plate (1), which cover layer (4) has a control window (41) in the area of the test section (34) of the test strip (3) of the at least one test strip receptacle (12), wherein the cover layer (4) has a sample application window (42) arranged at a distance from the control window (41), which allows for a sample application onto the sample receiving section (32) of at least one test strip (3).

8. Device according to claim 7, **characterised in that** at least two test strip receptacles (12) arranged in parallel to one another, each receiving one test strip (3), are arranged, which on one end lead into a common reservoir (13), in which the sample receiving sections (32) of the test strips (3) are arranged and above which the sample application window (42) of the cover layer (4) is positioned.

9. Device according to one of the previous claims, **characterised in that** the carrier plate (1) is made of cardboard, wherein existing test strip receptacles (12) and/or reservoirs (13) and/or sample carrier receptacles (14) are formed by impressions.

10. Device according to one of claims 1 to 8, **characterised in that** existing test strip receptacles (12) and/or reservoirs (13) and/or sample carrier receptacles (14) are formed by recesses (21) of an interlayer (2) arranged between cover film (4) and carrier plate (1).

11. Device according to one of the previous claims, **characterised in that** the cover layer (4) is formed of a film, which is preferably substantially two-dimensionally adhered to the carrier plate (1) and the test strip (3) received by the carrier plate (1) as well as to the DS sample carrier (5).

12. Device according to one of the previous claims, **characterised in that** two carrier plates (1) pivotably connected to one another are arranged, wherein one carrier plate is provided with at least one test strip receptacle (12) and the other carrier plate (1) abutting against it is provided with at least one sample carrier receptacle (14).

13. Device according to claim 12, **characterised in that** the at least one test strip receptacle (12) as well as the at least one sample carrier receptacle (14) are respectively arranged on one half of the surface of the carrier plate (1) in such a manner that in the folded state, the half of a carrier plate (1) provided with test strip receptacles (12) abuts against the half not provided with any sample carrier receptacle (14) of the other carrier plate (1) abutting against it.

14. Device according to one of the previous claims, **characterised in that** at least one reservoir (13) and/or one sample carrier receptacle (14) are at least partially enclosed by point-shaped and/or linear projections projecting from the carrier plate (1).

## Revendications

1. Dispositif de contrôle et d'affichage de la présence ou de l'absence d'analytes dans un échantillon d'analyse ou de liquides provenant de l'organisme humain ou d'un organisme animal, comprenant une plaque support avec au moins un réceptacle pour bandelette réactive dans lequel est disposée une bandelette réactive présentant un segment de réception de l'échantillon et un segment de test, **caractérisé en ce que** la plaque support comprend au moins un logement support (14) d'échantillon à distance du réceptacle (12) de bandelette réactive, sachant que le dispositif contient en outre un support d'échantillon « dried spot » (support d'échantillon DS) (5) qui est logé dans le logement support (14) d'échantillon.

2. Dispositif selon la revendication 1, **caractérisé en ce que** le support d'échantillon DS (5) présente au moins trois, de préférence quatre, zones d'application (15) de l'échantillon configurées de préférence circulaires.

3. Dispositif selon la revendication 1 ou 2, **caractérisé en ce que** le support d'échantillon DS (5) est fabriqué en cellulose.

4. Dispositif selon la revendication 3, **caractérisé en ce que** le support d'échantillon DS (5) est configuré en forme de papier filtre.

5. Dispositif selon l'une des revendications 2 à 4, **caractérisé en ce que** le support d'échantillon DS (5) est obturé au moins localement au moyen d'une couche de couverture (4) reliée de manière non détachable avec la plaque support (1), laquelle couche de couverture (4) présente au moins un évidement (43) dans l'espace des zones (15) d'application de l'échantillon, évidement qui est obturé au moyen d'un couvercle détachable.

6. Dispositif selon la revendication 5, **caractérisé en ce que** le couvercle détachable est limité par une ligne de perforation ménagée dans la couche de couverture (4).

7. Dispositif selon l'une des revendications précédentes, **caractérisé en ce qu'**au moins un réceptacle (12) de bandelette réactive est obturé au moins localement via une couche de couverture (4) reliée de manière non détachable à la plaque support (1), laquelle couche de couverture (4) présente une fenêtre de contrôle (41) dans la zone du segment de test (34) de la bandelette réactive (3) d'au moins un réceptacle (12) de bandelette réactive, sachant que la couche de couverture (4) présente, à distance de la fenêtre de contrôle (41), une fenêtre (42) d'application de l'échantillon permettant d'appliquer l'échantillon sur le segment (32) recevant l'échantillon d'au moins une bandelette réactive (3).

8. Dispositif selon la revendication 7, **caractérisé en ce que** sont disposés réciproquement parallèles au moins deux réceptacles (12) pour bandelettes réactives recevant chacun une bandelette réactive (3), réceptacles qui à une extrémité aboutissent dans un réservoir commun (13) dans lequel sont disposés les segments (32), recevant l'échantillon, des bandelettes réactives (3) et au-dessus duquel est positionnée la fenêtre (42), d'application de l'échantillon, de la couche de couverture (4).

9. Dispositif selon l'une des revendications précédentes, **caractérisé en ce que** la plaque support (1) est fabriquée en carton, sachant que des réceptacles (12) pour bandelettes réactives et/ou des réservoirs (13) et/ou des logements (14) supports d'échantillon présents sont formés par des gaufrages.

10. Dispositif selon l'une des revendications 1 à 8, **caractérisé en ce que** des réceptacles (12) pour bandelettes réactives et/ou des réservoirs (13) et/ou des logements (14) supports d'échantillons présents sont formés par des évidements (21) dans une couche intermédiaire (2) disposée entre la feuille de couverture (4) et la plaque support (1).

11. Dispositif selon l'une des revendications précédentes, **caractérisé en ce que** la couche de couverture (4) est formée par un film qui de préférence est collé à plat contre la plaque support (1) et les bandelettes réactives (3) reçues par la plaque support (1) et qui est collé sur le support d'échantillon DS (Dried spot) (5).

12. Dispositif selon l'une des revendications précédentes, **caractérisé en ce que** sont disposées deux plaques supports (1) reliées l'une à l'autre de manière pivotante, sachant qu'une plaque support est munie d'au moins un réceptacle (12) pour bandelette réactive et que l'autre plaque support (1) appliquant contre celui-ci est munie d'au moins un logement (14) support d'échantillon.

13. Dispositif selon la revendication 12, **caractérisé en ce qu'**au moins un réceptacle (12) pour bandelette réactive ainsi qu'au moins un logement (14) support d'échantillon sont disposés chacun sur une moitié de la surface d'une plaque support (1), de sorte qu'à l'état replié la moitié respective, munie des réceptacles (12) pour bandelettes réactives, d'une plaque support (1) applique contre la moitié, ne présentant pas de logement (14) support d'échantillon, de l'autre plaque support (1) appliquant contre celle-ci.

14. Dispositif selon l'une des revendications précédentes, **caractérisé en ce qu'**au moins un réservoir (13) et/ou un logement (14) support d'échantillon est entouré au moins localement de protubérances en pointillés et/ou linéaires dépassant de la plaque support (1).
